# EUROPÄISCHE PATENTSCHRIFT

(11) **EP 1 585 540 B1**
(45) Veröffentlichungstag und Bekanntmachung des Hinweises auf die Patenterteilung: **27.04.2011**
(21) Anmeldenummer: 04703150.5
(22) Anmeldetag: 19.01.2004
(51) Int. Cl.: A61K 38/17, A61P 35/00

(54) **MEDIKAMENT UND VERWENDUNG ZUR TUMORTHERAPIE**
MEDICAMENT AND USE THEREOF FOR TUMOR THERAPY
MEDICAMENT ET SON UTILISATION POUR LA THERAPIE D'UNE TUMEUR

(30) Priorität: 21.01.2003 DE 10302422
(43) Veröffentlichungstag der Anmeldung: 19.10.2005
(73) Patentinhaber: responsif GmbH, 91056 Erlangen (DE)
(72) Erfinder: BERTLING, Wolf, 91056 Erlangen (DE)
(74) Vertreter: Gassner, Wolfgang
(86) Internationale Anmeldenummer: PCT/EP2004/000362
(87) Internationale Veröffentlichungsnummer: WO 2004/064855

(56) Entgegenhaltungen:
- DE-A- 19 541 284
- US-A1- 2002 192 162

## Beschreibung

Die Erfindung betrifft ein Medikament und eine Verwendung zur Tumortherapie.

Aus der DE 195 41 284 A1 ist es bekannt, dass Annexin V sich zur Therapie von Tumoren eignet. Das wird darauf zurückgeführt, dass durch die Gabe von Annexin V die Phosphatidylserin abhängige Phagozytose beeinflussbar ist.

Nach-dem Stand der Technik ist kein Medikament bekannt, dass eine wirksame Tumortherapie gewährleistet.

Aufgabe der Erfindung ist es, die Nachteile nach dem Stand der Technik zu beseitigen. Es sollen insbesondere ein Medikament und eine Verwendung zur Tumortherapie angegeben werden.

Diese Aufgabe wird durch die Merkmale der Ansprüche 1 und 15 gelöst. Zweckmäßige Ausgestaltungen der Erfindungen ergeben sich aus den Merkmalen der Ansprüche 2 bis 14 und 16 bis 30.

Nach Maßgabe der Erfindung wird ein Medikament zur Tumortherapie vorgeschlagen, bei dem in einer wirksamen Konzentration ein erstes und ein zweites Molekül enthalten sind, wobei das erste Molekül
a1) Annexin V oder ein damit weit gehend ähnliches Molekül,
   oder
a2) ein wirksames Fragment von Annexin V oder des damit weit gehend ähnlichen Moleküls ist,
und wobei das zweite Molekül
b1) ein Cytokin oder ein damit weit gehend ähnliches Molekül
   oder
b2) ein wirksames Fragment eines Cytokin öder des damit weit gehend ähnlichen Moleküls ist.

Es hat sich überraschenderweise gezeigt, dass eine kombinierte Verabreichung von Annexin V bzw. gleichwirkender ähnlicher Moleküle und eines Cytokins oder eines damit gleichwirkenden ähnlichen Moleküls sich hervorragend zur Therapie von Tumoren eignet. Es wird innerhalb kurzer Zeit, z. B. innerhalb weniger Tage, eine Verringerung-der-Tumormasse bis hin zum völligen Verschwinden des Tumors beobachtet.

Das vorgeschlagene Medikament ist auch dann noch wirksam, wenn das erste Molekül lediglich ein mit Annexin V oder dem Cytokin ähnliches Molekül oder ein wirksames Fragment von Annexin V bzw. dem Cytokin oder ein mit dem Fragment weit gehend ähnliches Molekül umfasst. - Ein Fragment ist insbesondere dann "wirksam", wenn es in Kombination mit dem jeweils anderen Molekül ein Abschmelzen eines behandelten Tumors bewirkt. Unter dem Begriff "ähnliche Moleküle" werden solche Moleküle verstanden, welche zu einem gewissen Grad eine Identität mit Annexin V bzw. dem Cytokin aufweisen und in Kombination mit dem jeweils anderen Molekül wirksam sind.

Unter dem Begriff "Cytokin" wird ein Protein verstanden, das von einer Zelle freigesetzt wird.und das Verhalten anderer Zellen beeinflusst.

Nach einer Ausgestaltung kann eine Aminosäuresequenz des ersten Moleküls der Aminosäuresequenz der SEQ ID Nr. 1 oder Nr. 2 entsprechen oder zumindest zu 50%, vorzugsweise zumindest zu 60%, besonders bevorzugt zumindest zu 70%, ganz besonders bevorzugt zumindest zu 80%, identisch damit sein. Die Ermittlung der Identität kann erfolgen z.B. nach dem Verfahren von Altschul, S. F. et al. (1997), Nucleic Acids Res. 25:3389-3402.

Unter dem Begriff "Identität" wird vorliegend das Ausmaß verstanden, zu welchem zwei Nukleotid- oder Aminosäuresequenzen invariant sind.

Bei der Aminosäuresequenz der SEQ ID Nr. 2 handelt es sich um eine N-terminale Deletionsmutante der Aminosäuresequenz der SEQ ID Nr. 1, der die acht Aminosäuren 3 bis 10, das heißt die Aminosäuren Lys Tyr Thr Arg Gly Thr Val Thr fehlen.

Zweckmäßigerweise ist das Annexin V nicht-humanes Annexin V, vorzugsweise das Annexin V des Huhns. Ein Vergleich der Aminosäuresequenz des Annexin V des Huhns mit menschlichem Annexin V ergibt, dass beide Proteine zu 78,2 % identisch sind. Das Annexin V des Huhnes besitzt einen theoretischen isoelektrischen Punkt (pI) von 5,60, während menschliches Annexin V einen theoretischen isoelektrischen Punkt von 4,94 aufweist. Die Sequenz des menschlichen Annexins kann unter der Zugriffsnummer PO8756 in der Protein Datenbank "SWISS-PROT" abgerufen werden.

Das Cytokin kann aus der folgenden Gruppe ausgewählt sein: Interleukin-2, Interleukin-6, Interleukin-7, Interleukin-12, GM-CSF, TNF-α, IL-1β.

Es hat sich als besonders wirkungsvoll erwiesen, dass in einer Verabreichung 0,05 bis 0,5 mg/g_{Tumorgewicht} an erstem Molekül in einer Verabreichungseinheit enthalten sind. Dabei kann eine Verabreichungseinheit 0,1 bis 2,5 mg, vorzugsweise 0,5 bis 2,0 mg, an erstem Molekül enthalten. Sie enthält vorzugsweise ferner 50.000 bis 1.000.000 International Units, vorzugsweise 300.000 bis 750.000 International Units an zweitem Molekül. Ferner sind das erste und das zweite Molekül zweckmäßigerweise in einer Injektionsflüssigkeit, vorzugsweise einer gepufferten Kochsalzlösung, aufgenommen. Das Volumen der Injektionsflüssigkeit kann 0,5 bis 50 ml, vorzugsweise 1 bis 10 ml, betragen.

Das Medikament kann neben dem Wirkstoff weiterhin menschliche Tumorzellen umfassen, wobei die Tumorzellen apoptotische und/oder nekrotische Tumorzellen sein können. Die Apoptose und/oder Nekrose der Tumorzellen kann dabei spontan aufgetreten oder induziert worden sein. Als Induktoren für die Apoptose und/oder Nekrose kommen Bestrahlung der Tumorzellen exvivo oder in-vivo oder ein Inkontaktbringen der Tumorzellen mit Zytostatika in Betracht. Es eignen sich in diesem Zusammenhang insbesondere Chemikalien wie H₂O₂ oder Staurosporin, Medikamente wie Kortikosteroide, Chemotherapeutika wie Doxorubicin, cis-Platin oder Hydroxy-Harnstoff, UVB- und UVC-Strahlung, sowie β-, γ- oder Röntgenstrahlung. Vorzugsweise werden die apoptotischen und/oder nekrotischen Tumorzellen des zu behandelnden Tumors mit dem Wirkstoff in Kontakt gebracht.

Nach weiterer Maßgabe der Erfindung ist eine Verwendung eines ersten Moleküls, nämlich
a1) Annexin V oder eines damit weit gehend ähnlichen Moleküls,
   oder
a2) eines wirksamen Fragments von Annexin V oder eines damit weit gehend ähnlichen Moleküls,
in Kombination mit einem zweiten Molekül, nämlich
b1) Interleukin-2 oder einem damit weit gehend ähnlichen Molekül
   oder
b2) einem wirksamen Fragment von Interleukin-2 oder des damit weit gehend ähnlichen Moleküls,
zur Tumortherapie vorgesehen.

Als besonders wirkungsvoll hat sich die Verwendung zur Behandlung von Tumorergüssen erwiesen. Bei dem Tumor kann es sich insbesondere um ein Mammakarzinom handeln.

Wegen der vorteilhaften Ausgestaltungen wird auf die vorhergehende Beschreibung verwiesen. Die darin erwähnten Merkmale können sinngemäß auch Ausgestaltungen des Medikaments sein.

Die Erfindung wird nachstehend anhand der Zeichnungen beispielhaft näher erläutert. Es zeigen:
- Fig. 1: die Expressionskinetik von Annexin V des Huhns in transformierten Escherichia Coli BL21 (DE3) anhand einer Polyacrylamid-Gelelektrophorese und
- Fig. 2: eine Polyacrylamid-Gelelektrophorese von Proben der einzelnen Reinigungsschritte von Annexin V des Huhns aus transformierten Escherichia Coli BL21 (DE3).

### A. Expression und Reinigung von Annexin V des Huhns

### 1. Expression von Annexin V des Huhns

Für die Expression von Annexin V des Huhns (cAnxV) wurde als Expressionsplasmid pDJ2-AnxV verwendet, dass neben dem cAnxV kodierenden Gen einen IPTG-induzierbaren tac-Promotor und eine Kanamycin-Resistenz-Kassette enthält. E. coli BL21 (DE3) wurde mit dem Expressionsplasmid transformiert und Expressionskinetiken durchgeführt. Dabei wurde 2xYT mit 50 mg/l Kanamycin als Nährmedium eingesetzt.

Für die Herstellung von cAnxV wurde eine 100 ml Vorkultur mit frisch transformierten E. coli BL21 (DE3) angeimpft und 8 h bei 37°C geschüttelt. 5 l Hauptkultur wurden mit 5 ml der Vorkultur versetzt und 16 bis 20 h bei 37°C geschüttelt. Auf einen Zusatz von IPTG wurde verzichtet, da in diesem Fall mit und ohne Induktion gleiche Expressionsausbeuten erzielt wurden. Die Zellen wurden anschließend durch Zentrifugation geerntet. Die Zellfeuchtmasse betrug 16 bis 21 g. Die Expressionskinetik ist in Fig. 1 dargestellt. Die Expressionskinetiken ergaben, dass sich E. coli BL21 (DE3) auch ohne Induktion mit IPTG für eine Expression von cAnxV in dem eingesetzten Medium eignet.

### 2. Reinigung von Annexin V des Huhns

Die gemäß Ziff. 1 erhaltenen Zellen wurden in einem Puffer A1 (20 mM Tris/HCl pH 7,5, 2 mM EDTA) resuspendiert und mittels Hochdruck (Gaulin) aufgeschlossen. Unlösliche Bestandteile der Aufschlusssuspension wurden durch hochtourige Zentrifugation entfernt. Der lösliche cAnxV-enthaltende Überstand wurde auf eine in Puffer A1 äquilibrierte Q-Sepharose-ff-Säule (Säule 1) (25 ml, amersham pharmacia, Freiburg) aufgetragen. Die Elution des Zielproteins erfolgte durch einen linearen NaCl-Gradienten. Die cAnxV-enthaltenden Fraktionen wurden gepoolt und gegen einen Puffer A2 (50 mM Na-Acetat pH 5,6) dialysiert. Das Dialysat wurde auf eine in A2 äquilibrierte Resource-S-Säule (Säule 2) (6 ml, amersham pharmacia, Freiburg) aufgetragen und cAnxV mit einem linearen NaCl-Gradienten eluiert. Die vereinigten cAnxV-enthaltenden Fraktionen wurden mittels Ultrafiltration (Pall Filtron, USA) konzentriert und auf eine in 10 mM Na-Phosphat pH 7,2, 140 mM NaCl äquilibrierte Superdex 200 pg-Säule (Säule 3) (amersham pharmacia, Freiburg) aufgetragen. Homogenes cAnxV wurde von der Säule eluiert. Aus 20 g Zellen (Feuchtmasse) wurden 30 mg cAnxV mit einer Reinheit, die 95 % überstieg, isoliert. Fig. 2 zeigt anhand einer Polyacrylamid-Gelelektrophorese die Ergebnisse der Reinigung unter Verwendung der Säulen 1 bis 3.

Alternativ kann anstelle der angegebenen Säulen für Säule 1 Q-Sepharose XL (amersham pharmacia, Freiburg), für Säule 2 SP-Sepharose HP (amersham pharmacia, Freiburg) und/oder für Säule 3 Sephacryl S200 HR (amersham pharmacia, Freiburg) verwendet werden.

Alternativ kann anstelle der mittels Säule 3 durchgeführten Größen-Ausschluss-Chromatografie (SEC) eine hydrophobe Säule eingesetzt werden. In diesem Fall werden die cAnxV-enthaltenden Fraktionen, die von Säule 2 eluieren, vereinigt und mit festem Ammoniumsulfat auf 1,5 M aufgestockt. Die Proteinlösung wird auf eine Phenylsepharose ff-Säule (15 ml, amersham pharmacia, Freiburg) aufgetragen und cAnxV mit einem linearen Gradienten von 1,5 bis 0 M Ammoniumsulfat eluiert.

### B. Tumortherapie

Einem Patienten wurden in einem individuellen Heilversuch in 1 ml gepufferter Kochsalzlösung aufgenommenes Annexin V gemäß Sequenzprotokoll SEQ ID NO: 1 zusammen mit 500.000 Interntional Units Interleukin-2 in ein etwa 2 cm großes Melanom injiziert. Es zeigt sich bereits nach wenigen Tagen ein deutliches Abschmelzen des Melanoms.

Nach einem weiteren Therapieverfahren ist es auch möglich, zunächst dem Patienten Tumorzellen zu entnehmen. Die entnommenen Tumorzellen werden mechanisch dissoziiert; es wird die Zellenzahl bestimmt. Anschließend werden die Zellen mit 100 Gray bestahlt, so dass die Tumorzellen in apoptotische oder nekrotische Tumorzellen überführt werden. 10 x 10⁶ der apoptotischen oder nekrotischen Tumorzellen werden anschließend mit einer gepufferten Kochsalzlösung gemischt, die 1 mg Annexin V gemäß Sequenzprotokoll SEQ.l oder SEQ.2 enthält. Es erfolgt anschließend eine Inkubation. Unmittelbar vor der Injektion werden 500.000 International Units Interleukin-2 hinzugfügt. Die die apoptotischen Tumorzellen, Annexin V und Interleukin-2 enthaltende Mischung wird dann dem-Patienten intradermal oder subkutan injiziert. Auch dabei wird bereits nach wenigen Tagen ein signifikantes Abschmelzen des behandelten Tumors beobachtet.

Zur Steigerung der Effizienz des vorgenannten Therapieverfahrens und zur Bekämpfung eines Rezidivs kann die Injektion z. B. am Tag 21, 42 und an späteren Tagen oder in der zweiten, dritten und sechsten Woche wiederholt werden.

Zum Nachweis der besonderen Wirksamkeit einer kombinierten Verabreichung von Cytokin und Annexin V werden die überstände von peritonealen Makrophagen sowie von aus Knochenmark gewonnenen dentritischen Zellen jeweils für 24 Stunden mit einem Medium inkubiert. Abschließend werden jeweils die Mengen (in pg/ml) an freigesetzten Cytokinen, nämlich TNF-α, IL-1β, mittels ELISA ermittelt. Die Versuche werden unter identischen Bedingungen wiederholt, wobei das Medium zusammen mit bestrahlten Tumorzellen (ITC), mit Annexin V (AxV) sowie mit bestrahlten Tumorzellen und Annexin V inkubiert wird. Bei der Co-Inkubation hat das Verhältnis von ITC:Phagozyten 5:1 betragen. Die erhaltenen Ergebnisse sind nachdem Students t Test ausgewertet worden, wobei *= p < 0,01; ** = p < 0,005.

In der nachfolgende Tabelle sind die gewonnenen Ergebnisse vergleichend dargestellt.

| | Makrophagen | | | | Dendritische Zellen | | | |
|---|---|---|---|---|---|---|---|---|
| Cytokin (pg/ml) | Medium | | Medium + AxV | | Medium | | Medium + AxV | |
| | / | ITC | / | ITC | / | ITC | / | ITC |
| TNF-α | 125±15 | 335±30 | 117±24 | 978±48** | 267±137 | 392±79 | 245±121 | 426±78 |
| IL-1β | 21±5 | 44±7 | 14±1 | 322±85* | 10±4 | 46±4 | 8±3 | 47±3 |

| | | | | | | | | |
|---|---|---|---|---|---|---|---|---|
| * *P* <0.05 ** *P* <0.01 | | | | | | | | |

Wie aus den Ergebnissen deutlich hervorgeht, ist nach Co-Inkubation mit bestrahlten Tumorzellen die Sezernierung postinflammatorischer Cytokine, nämlich TNF-α, IL-1β, durch Makrophagen deutlich erhöht. Bei einer Co-Inkubation von Makrophagen mit bestrahlten und mit Annexin V behandelten Tumorzellen wird dieser Effekt drastisch verstärkt.

## Patentansprüche

1. Medikament zur Anwendung in der Tumortherapie, bei dem in einer wirksamen Konzentration ein erstes und ein zweites Molekül enthalten sind, wobei das erste Molekül
a1) Annexin V oder ein damit weit gehend ähnliches Molekül,
oder
a2) ein wirksames Fragment von Annexin V oder des damit weit gehend ähnlichen Moleküls ist,
wobei die Aminosäuresequenz des ersten Moleküls der Aminosäuresequenz der SEQ ID Nr. 1 oder Nr. 2 entspricht oder zumindest zu 50% identisch damit ist,
und wobei das zweite Molekül
b1) ein Cytokin oder ein damit weit gehend ähnliches Molekül
oder
b2) ein wirksames Fragment eines Cytokins oder des damit weit gehend ähnlichen Moleküls ist.

2. Medikament zur Anwendung nach Anspruch 1, wobei die Aminosäuresequenz des ersten Moleküls zumindest zu 60%, bevorzugt zu zumindest 70%, besonders bevorzugt zumindest zu 80% identisch ist mit der Aminosäuresequenz der SEQ ID Nr. 1 oder Nr. 2.

3. Medikament zur Anwendung nach einem der vorhergehenden Ansprüche, wobei das Annexin V nicht-humanes Annexin V ist.

4. Medikament zur Anwendung nach einem der vorhergehenden Ansprüche, wobei das nicht-humane Annexin V das Annexin V des Huhns ist.

5. Medikament zur Anwendung nach einem der vorhergehenden Ansprüche, wobei das Cytokin aus der folgenden Gruppe ausgewählt ist: Interleukin-2, Interleukin-6, Interleukin-7, Interleukin-12, GM-CSF, TNF-α, IL-1β.

6. Medikament zur Anwendung nach einem der vorhergehenden Ansprüche, wobei 0,05 bis 0,5mg/G_{Tumorgewicht} an erstem Molekül in einer Verabreichungseinheit enthalten sind.

7. Medikament zur Anwendung nach einem der vorhergehenden Ansprüche, wobei eine Verabreichungseinheit 0,1 bis 2,5 mg, vorzugsweise 0,5 bis 2,0 mg, an erstem Molekül enthält.

8. Medikament zur Anwendung nach einem der vorhergehenden Ansprüche, wobei eine Verabreichungseinheit 50.000 bis 1.000.000 International Units, vorzugsweise 300.000 bis 750.000 International Units, an zweitem Molekül enthält.

9. Medikament zur Anwendung nach einem der vorhergehenden Ansprüche, wobei das erste und das zweite Molekül in einer Injektionsflüssigkeit, vorzugsweise einer gepufferten Kochsalzlösung, aufgenommen sind.

10. Medikament zur Anwendung nach einem der vorhergehenden Ansprüche, wobei das Volumen der Injektionsflüssigkeit 0,5 bis 50 ml, vorzugsweise 1 bis 10 ml, beträgt.

11. Medikament zur Anwendung nach einem der vorhergehenden Ansprüche, wobei es apoptotische und/oder nekrotische Tumorzellen umfasst.

12. Medikament zur Anwendung nach einem der vorhergehenden Ansprüche, wobei es weiterhin menschliche Tumorzellen umfasst.

13. Medikament zur Anwendung nach einem der vorhergehenden Ansprüche 11-12, wobei die Tumorzellen apoptotische und/oder nekrotische Tumorzellen des zu behandelnden Tumors sind.

14. Medikament zur Anwendung nach einem der vorhergehenden Ansprüche 11-13, wobei die Tumorzellen mit dem Protein in Kontakt sind.

15. Erstes Molekül, nämlich
a1) Annexin V oder ein damit weit gehend ähnliches Molekül
oder
a2) ein wirksames Fragment von Annexin V oder des damit weit gehend ähnlichen Moleküls,
wobei die Aminosäuresequenz des ersten Moleküls der Aminosäuresequenz der SEQ ID Nr. 1 oder Nr. 2 entspricht oder zumindest zu 50% identisch damit ist,
in Kombination mit einem zweiten Molekül, nämlich
b1) einem Cytokin oder einem damit weit gehend ähnlichen Molekül
oder
b2) einem wirksamen Fragment eines Cytokins oder des damit weit gehend ähnlichen Moleküls,
zur Anwendung in der Tumortherapie.

16. Kombination zur Anwendung in der Tumortherapie nach Anspruch 15, wobei der Tumor ein Tumorerguss ist.

17. Kombination zur Anwendung in der Tumortherapie nach Anspruch 15 oder 16, wobei der Tumor ein Mammakarzinom ist.

18. Kombination zur Anwendung in der Tumortherapie nach einem der Ansprüche 15 bis 17, wobei die Aminosäuresequenz des ersten Moleküls zumindest zu 60%, bevorzugt zu zumindest 70%, besonders bevorzugt zumindest zu 80%, identisch mit der Aminosäuresequenz der SEQ ID Nr. 1 oder Nr. 2 ist.

19. Kombination zur Anwendung in der Tumortherapie nach einem der Ansprüche 15 bis 18, wobei das Annexin V nicht-humanes Annexin V ist.

20. Kombination zur Anwendung in der Tumortherapie nach einem der Ansprüche 15 bis 19, wobei das nicht-humane Annexin V das Annexin V des Huhns ist.

21. Kombination zur Anwendung in der Tumortherapie nach einem der Ansprüche 15 bis 20, wobei das Cytokin aus der folgenden Gruppe ausgewählt ist: Interleukin-2, Interleukin-6, Interleukin-7, Interleukin-12, GM-CSF, TNF-α, IL-1β.

22. Kombination zur Anwendung in der Tumortherapie nach einem der Ansprüche 15 bis 21, wobei 0,05 bis 0,5 mg/g_{Tumorgewicht} an erstem Molekül V in einer Verabreichungseinheit enthalten ist.

23. Kombination zur Anwendung in der Tumortherapie nach einem der Ansprüche 15 bis 22, wobei eine Verabreichungseinheit 0,1 bis 2,5 mg, vorzugsweise 0,5 bis 2,0 mg, an erstem Molekül enthält.

24. Kombination zur Anwendung in der Tumortherapie nach einem der Ansprüche 15 bis 23, wobei eine Verabreichungseinheit 50.000 bis 1.000.000 International Units, vorzugsweise 300.000 bis 750.000 International Units, an zweitem Molekül enthält.

25. Kombination zur Anwendung in der Tumortherapie nach einem der Ansprüche 15 bis 24, wobei das erste und das zweite Molekül in einer Injektionsflüssigkeit, vorzugsweise einer gepufferten Kochsalzlösung, aufgenommen sind.

26. Kombination zur Anwendung in der Tumortherapie nach einem der Ansprüche 15 bis 25, wobei das Volumen der Injektionsflüssigkeit 0,5 bis 50 ml, vorzugsweise 1 bis 10 ml, beträgt.

27. Kombination zur Anwendung in der Tumortherapie nach einem der Ansprüche 15 bis 26, wobei die Kombination apoptotische und/oder nekrotische Tumorzellen umfasst.

28. Kombination zur Anwendung in der Tumortherapie nach einem der Ansprüche 15 bis 27, wobei die Kombination weiterhin menschliche Tumorzellen umfasst.

29. Kombination zur Anwendung in der Tumortherapie nach einem der Ansprüche 27 bis 28, wobei die Tumorzellen apoptotische und/oder nekrotische Tumorzellen des zu behandelnden Tumors sind.

30. Kombination zur Anwendung in der Tumortherapie nach einem der Ansprüche 27 bis 29, wobei die Tumorzellen mit dem Protein in Kontakt sind.

## Claims

1. Medicament for the use in tumor therapy wherein a first and a second molecule are contained in an effective concentration, wherein the first molecule is
a1) Annexin V or a molecule which is largely similar thereto,
or
a2) an effective fragment of Annexin V or of the molecule which is largely similar thereto,
wherein the amino acid sequence of the first molecule corresponds to the amino acid sequence of the SEQ ID no. 1 or no. 2 or is at least 50% identical thereto,
and wherein the second molecule is
b1) a cytokine or a molecule which is largely similar thereto
or
b2) an effective fragment of a cytokine or of the molecule which is largely similar thereto.

2. Medicament for the use as defined in claim 1, wherein the amino acid sequence of the first molecule is identical to the amino acid sequence of SEQ ID no. 1 or no. 2 by at least 60%, preferably by at least 70%, particularly preferably by at least 80%.

3. Medicament for the use as defined in one of the preceding claims, wherein the Annexin V is a non-human Annexin V.

4. Medicament for the use as defined in one of the preceding claims, wherein the non-human Annexin V is the Annexin V of the chicken.

5. Medicament for the use as defined in one of the preceding claims, wherein the cytokine is selected from the following group: Interleukin-2, Interleukin-6, Interleukin-7, Interleukin-12, GM-CSF, TNF-α, IL-1β.

6. Medicament for the use as defined in one of the preceding claims, wherein 0.05 to 0.5 mg/g_{Tumorweight} on the first molecule are contained in one unit of administration.

7. Medicament for the use as defined in one of the preceding claims, wherein one unit of administration contains 0.1 to 2.5 mg, preferably 0.5 to 2.0 mg on the first molecule.

8. Medicament for the use as defined in one of the preceding claims, wherein one unit of administration contains 50,000 to 1,000,000 International Units, preferably 300,000 to 750,000 International Units on the second molecule.

9. Medicament for the use as defined in one of the preceding claims, wherein the first and the second molecule are held in an injection fluid, preferably in a buffered saline solution.

10. Medicament for the use as defined in one of the preceding claims, wherein the volume of the injection fluid is 0.5 to 50 ml, preferably 1 to 10 ml.

11. Medicament for the use as defined in one of the preceding claims, wherein it includes apoptotic and/or necrotic tumor cells.

12. Medicament for the use as defined in one of the preceding claims, wherein it further includes human tumor cells.

13. Medicament for the use as defined in one of the preceding claims 11 to 12, wherein the tumor cells are apoptotic and/or necrotic tumor cells of the tumor to be treated.

14. Medicament for the use as defined in one of the preceding claims 11 to 13, wherein the tumor cells are in contact with the protein.

15. First molecule, namely
a1) Annexin V or a molecule which is largely similar thereto
or
a2) an effective fragment of Annexin V or of the molecule which is largely similar thereto,
wherein the amino acid sequence of the first molecule corresponds to the amino acid sequence of the SEQ ID no. 1 or no. 2 or is at least 50% identical thereto,
in combination with a second molecule, namely
b1) a cytokine or a molecule which is largely similar thereto
or
b2) an effective fragment of a cytokine or of the molecule which is largely similar thereto,
for the use in tumor therapy.

16. Combination for the use in tumor therapy as defined in claim 15, wherein the tumor is a tumor effusion.

17. Combination for the use in tumor therapy as defined in claim 15 or 16, wherein the tumor is a mamma carcinoma.

18. Combination for the use in tumor therapy as defined in one of the claims 15 to 17, wherein the amino acid sequence of the first molecule is identical to the amino acid sequence of SEQ ID no. 1 or no. 2 by at least 60% thereto, preferably by at least 70%, particularly preferably by at least 80%.

19. Combination for the use in tumor therapy as defined in one of the claims 15 to 18, wherein the Annexin V is non-human Annexin V.

20. Combination for the use in tumor therapy as defined in one of the claims 15 to 19 wherein the non-human Annexin V is the Annexin V of the chicken.

21. Combination for the use in tumor therapy as defined in one of the claims 15 to 20, wherein the cytokine is selected from the following group: Interleukin-2, Interleukin-6, Interleukin-7, Interleukin-12, GM-CSF, TNF-α, IL-1β.

22. Combination for the use in tumor therapy as defined in one of the claims 15 to 21, wherein one administration unit contains 0.05 to 0.5 mg/g_{Tumorweight} on the first molecule.

23. Combination for the use in tumor therapy as defined in one of the claims 15 to 22, wherein one unit of administration contains 0.1 to 2.5 mg, preferably 0.5 to 2.0 mg on the first molecule.

24. Combination for the use in tumor therapy as defined in one of the claims 15 to 23, wherein one unit of administration contains 50,000 to 1,000,000 International Units, preferably 300,000 to 750,000 International Units on the second molecule.

25. Combination for the use in tumor therapy as defined in one of the claims 15 to 24, wherein the first and the second molecule are contained in an injection fluid, preferably in a buffered saline solution.

26. Combination for the use in tumor therapy as defined in one of the claims 15 to 25, wherein the volume of the injection fluid is 0.5 to 50 ml, preferably 1 to 10 ml.

27. Combination for the use in tumor therapy as defined in one of the claims 15 to 26, wherein the combination includes apoptotic and/or necrotic tumor cells.

28. Combination for the use in tumor therapy as defined in one of the claims 15 to 27, wherein the combination further includes human tumor cells.

29. Combination for the use in tumor therapy as defined in one of the claims 27 to 28, wherein the tumor cells are apoptotic and/or necrotic tumor cells of the tumor to be treated.

30. Combination for the use in tumor therapy as defined in one of the claims 27 to 29, wherein the tumor cells are in contact with the protein.

## Revendications

1. Médicament pour l'utilisation dans la thérapie de tumeurs, où une première et une deuxième molécule sont contenues dans une concentration efficace, la première molécule étant
a1) de l'annexine V ou une molécule largement similaire à celle-ci,
ou
a2) un fragment efficace d'annexine V ou de la molécule largement similaire à celle-ci,
la séquence d'acides aminés de la première molécule correspondant à la séquence d'acides aminés de la SEQ ID N° 1 ou N° 2 ou étant au moins identique à 50% à celle-ci,
et la deuxième molécule étant
b1) une cytokine ou une molécule largement similaire à celle-ci
ou
b2) un fragment efficace d'une cytokine ou de la molécule largement similaire à celle-ci.

2. Médicament pour l'utilisation selon la revendication 1, la séquence d'acides aminés de la première molécule étant identique au moins à 60 %, de préférence au moins à 70 %, en particulier de préférence au moins à 80 %, à la séquence d'acides aminés de la SEQ ID N° 1 ou N° 2.

3. Médicament pour l'utilisation selon l'une des revendications précédentes, l'annexine V étant de l'annexine V non humaine.

4. Médicament pour l'utilisation selon l'une des revendications précédentes, l'annexine V non humaine étant l'annexine V du poulet.

5. Médicament pour l'utilisation selon l'une des revendications précédentes, la cytokine étant sélectionnée parmi le groupe suivant : interleukine-2, interleukine-6, interleukine-7, interleukine-12, GM-CSF, TNF-α, IL-1β.

6. Médicament pour l'utilisation selon l'une des revendications précédentes, où 0, 05 à 0, 5 mg/g_{poids tumoral} sur la première molécule est compris dans une unité d'administration.

7. Médicament pour l'utilisation selon l'une des revendications précédentes, où une unité d'administration contient de 0,1 à 2,5 mg, de préférence de 0,5 à 2,0 mg, sur la première molécule.

8. Médicament pour l'utilisation selon l'une des revendications précédentes, où une unité d'administration contient de 50 000 à 1 000 000 unités internationales, de préférence de 300 000 à 750 000 unités internationales, sur la deuxième molécule.

9. Médicament pour l'utilisation selon l'une des revendications précédentes, où la première et la deuxième molécule sont absorbées dans un liquide d'injection, de préférence dans une solution saline tamponnée.

10. Médicament pour l'utilisation selon l'une des revendications précédentes, où le volume du liquide d'injection est de 0,5 à 50 ml, de préférence de 1 à 10 ml.

11. Médicament pour l'utilisation selon l'une des revendications précédentes, comprenant des cellules tumorales apoptotiques et/ou nécrotiques.

12. Médicament pour l'utilisation selon l'une des revendications précédentes, comprenant en outre des cellules tumorales humaines.

13. Médicament pour l'utilisation selon l'une des revendications précédentes 11 à 12, les cellules tumorales étant des cellules tumorales apoptotiques et/ou nécrotiques de la tumeur à traiter.

14. Médicament pour l'utilisation selon l'une des revendications précédentes 11 à 13, les cellules tumorales étant en contact avec la protéine.

15. Première molécule, à savoir
a1) de l'annexine V ou une molécule largement similaire à celle-ci
ou
a2) un fragment efficace d'annexine V ou de la molécule largement similaire à celle-ci,
où la séquence d'acides aminés de la première molécule correspond à la séquence d'acides aminés de la SEQ ID N° 1 ou N° 2 ou est au moins à 50 % identique à celle-ci,
en combinaison avec une deuxième molécule, à savoir
b1) une cytokine ou une molécule largement similaire à celle-ci
ou
b2) un fragment efficace d'une cytokine ou de la molécule largement similaire à celle-ci,
pour l'utilisation dans la thérapie de tumeurs.

16. Combinaison pour l'utilisation dans la thérapie de tumeurs selon la revendication 15, où la tumeur est un épanchement tumoral.

17. Combinaison pour l'utilisation dans la thérapie de tumeurs selon la revendication 15 ou 16, ou la tumeur est un carcinome mammaire.

18. Combinaison pour l'utilisation dans la thérapie de tumeurs selon l'une des revendications 15 à 17, où la séquence d'acides aminés de la première molécule est au moins à 60 %, de préférence au moins à 70 %, en particulier de préférence au moins à 80 %, identique à la séquence d'acides aminés de la SEQ ID N° 1 ou N° 2.

19. Combinaison pour l'utilisation dans la thérapie de tumeurs selon l'une des revendications 15 à 18, où l'annexine V est une annexine V non humaine.

20. Combinaison pour l'utilisation dans la thérapie de tumeurs selon l'une des revendications 15 à 19, où l'annexine V non humaine est l'annexine V du poulet.

21. Combinaison pour l'utilisation dans la thérapie de tumeurs selon l'une des revendications 15 à 20, où la cytokine est sélectionnée parmi le groupe suivant : interleukine-2, interleukine-6, interleukine-7, interleukine-12, GM-CSF, TNF-α, IL-1β.

22. Combinaison pour l'utilisation dans la thérapie de tumeurs selon l'une des revendications 15 à 21, où 0,05 à 0,5 mg/g_{poids tumoral} sur la première molécule V est compris dans une unité d'administration.

23. Combinaison pour l'utilisation dans la thérapie de tumeurs selon l'une des revendications 15 à 22, où une unité d'administration contient 0,1 à 2,5 mg, de préférence 0,5 à 2,0 mg, sur la première molécule.

24. Combinaison pour l'utilisation dans la thérapie de tumeurs selon l'une des revendications 15 à 23, où une unité d'administration contient de 50 000 à 1 000 000 unités internationales, de préférence de 300 000 à 750 000 unités internationales, sur la deuxième molécule.

25. Combinaison pour l'utilisation dans la thérapie de tumeurs selon l'une des revendications 15 à 24, où la première et la deuxième molécule sont absorbées dans un liquide d'injection, de préférence dans une solution saline tamponnée.

26. Combinaison pour l'utilisation dans la thérapie de tumeurs selon l'une des revendications 15 à 25, où le volume du liquide d'injection est de 0,5 à 50 ml, de préférence de 1 à 10 ml.

27. Combinaison pour l'utilisation dans la thérapie de tumeurs selon l'une des revendications 15 à 26, où la combinaison contient des cellules tumorales apoptotiques et/ou nécrotiques.

28. Combinaison pour l'utilisation dans la thérapie de tumeurs selon l'une des revendications 15 à 27, où la combinaison comprend en outre des cellules tumorales humaines.

29. Combinaison pour l'utilisation dans la thérapie de tumeurs selon l'une des revendications 27 à 28, où les cellules tumorales sont des cellules tumorales apoptotiques et/ou nécrotiques de la tumeur à traiter.

30. Combinaison pour l'utilisation dans la thérapie de tumeurs selon l'une des revendications 27 à 29, où les cellules tumorales sont en contact avec la protéine.


### SEQUENZPROTOKOLL

<110> november Aktiengesellschaft Gesellschaft für Molekulare Medizin
<120> Medikament und Verwendung zur Tumortherapie
<130> 411814GA
<140>
   <141>
<160> 2
<170> PatentIn Ver. 2.1
<210> 1
   <211> 321
   <212> PRT
   <213> Gallus gallus
<300>
<400> 1
   Met Ala Lys Tyr Thr Arg Gly Thr Val Thr Ala Phe Ser Pro Phe Asp
    1               5                  10                  15
   Ala Arg Ala Asp Ala Glu Ala Leu Arg Lys Ala Met Lys Gly Met Gly
               20                  25                  30
   Thr Asp Glu Glu Thr Ile Leu Lys Ile Leu Thr Ser Arg Asn Asn Ala
           35                  40                  45
   Gln Arg Gln Glu Ile Ala Ser Ala Phe Lys Thr Leu Phe Gly Arg Asp
       50                  55                  60
   Leu Val Asp Asp Leu Lys Ser Glu Leu Thr Gly Lys Phe Glu Thr Leu
   65                  70                  75                  80
   Met Val Ser Leu Met Arg Pro Ala Arg Ile Phe Asp Ala His Ala Leu
                   85                  90                  95
   Lys His Ala Ile Lys Gly Ala Gly Thr Asn Glu Lys Val Leu Thr Glu
               100                 105                 110
   Ile Leu Ala Ser Arg Thr Pro Ala Glu Val Gln Asn Ile Lys Gln Val
           115                 120                 125
   Tyr Met Gln Glu Tyr Glu Ala Asn Leu Glu Asp Lys Ile Thr Gly Glu
       130                 135                 140
   Thr Ser Gly His Phe Gln Arg Leu Leu Val Val Leu Leu Gln Ala Asn
   145                 150                 155                 160
   Arg Asp Pro Asp Gly Arg Val Asp Glu Ala Leu Val Glu Lys Asp Ala
                   165                 170                 175
   Gln Val Leu Phe Arg Ala Gly Glu Leu Lys Trp Gly Thr Asp Glu Glu
               180                 185                 190
   Thr Phe Ile Thr Ile Leu Gly Thr Arg Ser Val Ser His Leu Arg Arg
           195                 200                 205
   Val Phe Asp Lys Tyr Met Thr Ile Ser Gly Phe Gln Ile Glu Glu Thr
       210                 215                 220
   Ile Asp Arg Glu Thr Ser Gly Asp Leu Glu Lys Leu Leu Leu Ala Val
   225                 230                 235                 240
   Val Lys Cys Ile Arg Ser Val Pro Ala Tyr Phe Ala Glu Thr Leu Tyr
                   245                 250                 255
   Tyr Ser Met Lys Gly Ala Gly Thr Asp Asp Asp Thr Leu Ile Arg Val
               260                 265                 270
   Met Val Ser Arg Ser Glu Ile Asp Leu Leu Asp Ile Arg His Glu Phe
           275                 280                 285
   Arg Lys Asn Phe Ala Lys Ser Leu Tyr Gln Met Ile Gln Lys Asp Thr
       290                 295                 300
   Ser Gly Asp Tyr Arg Lys Ala Leu Leu Leu Leu Cys Gly Gly Asp Asp
   305                 310                 315                 320
   Glu
<210> 2
   <211> 313
   <212> PRT
   <213> Gallus gallus
<400> 2
   Met Ala Ala Phe Ser Pro Phe Asp Ala Arg Ala Asp Ala Glu Ala Leu
    1               5                  10                  15
   Arg Lys Ala Met Lys Gly Met Gly Thr Asp Glu Glu Thr Ile Leu Lys
               20                  25                  30
   Ile Leu Thr Ser Arg Asn Asn Ala Gln Arg Gln Glu Ile Ala Ser Ala
           35                  40                  45
   Phe Lys Thr Leu Phe Gly Arg Asp Leu Val Asp Asp Leu Lys Ser Glu
       50                  55                  60
   Leu Thr Gly Lys Phe Glu Thr Leu Met Val Ser Leu Met Arg Pro Ala
   65                  70                  75                  80
   Arg Ile Phe Asp Ala His Ala Leu Lys His Ala Ile Lys Gly Ala Gly
                   85                  90                  95
   Thr Asn Glu Lys Val Leu Thr Glu Ile Leu Ala Ser Arg Thr Pro Ala
               100                 105                 110
   Glu Val Gln Asn Ile Lys Gln Val Tyr Met Gln Glu Tyr Glu Ala Asn
           115                 120                 125
   Leu Glu Asp Lys Ile Thr Gly Glu Thr Ser Gly His Phe Gln Arg Leu
       130                 135                 140
   Leu Val Val Leu Leu Gln Ala Asn Arg Asp Pro Asp Gly Arg Val Asp
   145                 150                 155                 160
   Glu Ala Leu Val Glu Lys Asp Ala Gln Val Leu Phe Arg Ala Gly Glu
                   165                 170                 175
   Leu Lys Trp Gly Thr Asp Glu Glu Thr Phe Ile Thr Ile Leu Gly Thr
               180                 185                 190
   Arg Ser Val Ser His Leu Arg Arg Val Phe Asp Lys Tyr Met Thr Ile
           195                 200                 205
   Ser Gly Phe Gln Ile Glu Glu Thr Ile Asp Arg Glu Thr Ser Gly Asp
       210                 215                 220
   Leu Glu Lys Leu Leu Leu Ala Val Val Lys Cys Ile Arg Ser Val Pro
   225                 230                 235                 240
   Ala Tyr Phe Ala Glu Thr Leu Tyr Tyr Ser Met Lys Gly Ala Gly Thr
                   245                 250                 255
   Asp Asp Asp Thr Leu Ile Arg Val Met Val Ser Arg Ser Glu Ile Asp
               260                 265                 270
   Leu Leu Asp Ile Arg His Glu Phe Arg Lys Asn Phe Ala Lys Ser Leu
           275                 280                 285
   Tyr Gln Met Ile Gln Lys Asp Thr Ser Gly Asp Tyr Arg Lys Ala Leu
       290                 295                 300
   Leu Leu Leu Cys Gly Gly Asp Asp Glu
   305                 310